# EUROPÄISCHE PATENTSCHRIFT

(11) **EP 3 856 272 B1**
(45) Veröffentlichungstag und Bekanntmachung des Hinweises auf die Patenterteilung: **25.01.2023**
(21) Anmeldenummer: 20720757.2
(22) Anmeldetag: 09.04.2020
(51) Int. Cl.: A61M 1/02, A61M 1/36, B04B 5/04

(54) **BLUTSEPARATIONSVORRICHTUNG**
BLOOD-SEPARATING DEVICE
DISPOSITIF SÉPARATEUR DE SANG

(30) Priorität: 12.04.2019 DE 102019109749
(43) Veröffentlichungstag der Anmeldung: 04.08.2021
(73) Patentinhaber: Lmb Technologie GmbH, 85445 Schwaig (DE)
(72) Erfinder: JENTSCH, Klaus, 85445 Schwaig (DE)
(74) Vertreter: Winter, Brandl - Partnerschaft mbB
(86) Internationale Anmeldenummer: PCT/EP2020/060212
(87) Internationale Veröffentlichungsnummer: WO 2020/208166

(56) Entgegenhaltungen:
- WO-A1-92/00145
- CN-A- 108 578 801
- US-A1- 2002 151 423
- US-A1- 2006 122 048
- US-A1- 2012 289 926

## Beschreibung

Die vorliegende Erfindung betrifft eine Blutseparationsvorrichtung zum Separieren von Blut unter Einfluss einer während eines Zentrifugiervorgangs in einer Zentrifuge erzeugten Zentrifugalkraft. Die vorliegende Erfindung betrifft insbesondere eine Blutseparationsvorrichtung, bei der sich die Blutkomponenten nach dem Zentrifugiervorgang in getrennten Behältnissen befinden.

Es ist bekannt, dass in einem Blutbeutel aufgenommenes Blut unter Einfluss einer während eines Zentrifugiervorgangs erzeugten Zentrifugalkraft in einzelne Komponenten entsprechend dem spezifischen Gewicht der Komponenten in Richtung der Zentrifugalkraft aufgetrennt wird. Anschließend ist es erforderlich, die einzelnen Komponenten jeweils in ein dafür vorgesehenes Behältnis zu überführen, so dass die Blutkomponenten einzeln für Untersuchungen und/oder therapeutische Zwecke zur Verfügung stehen.

Bei konventionellen Arbeitsverfahren wird daher das Blut zuerst zentrifugiert, so dass die einzelnen Blutkomponenten in Schichten getrennt im Blutbeutel vorhanden sind. Anschließend werden der Blutbeutel und dazugehörige Komponentenbeutel aus der Zentrifuge genommen und in eine separate Blutseparationsvorrichtung eingebracht, in der Blutkomponenten aus dem Blutbeutel in die Komponentenbeutel überführt werden. Bei diesem Vorgehen sind jedoch mehrere zeitaufwändige Arbeitsschritte erforderlich, so dass sich die einzelnen Blutkomponenten in der Zeit zwischen dem Zentrifugieren und dem Überführen in die dafür vorgesehenen Komponentenbeutel unter Verwendung der separaten Blutseparationsvorrichtung erneut vermischen.

Aus diesem Grund sind im Stand der Technik Arbeitsverfahren, wie beispielsweise aus DE 29 38 367 A1, bekannt, bei denen das Blut während eines Zentrifugiervorgangs im Blutbeutel in die einzelnen Blutkomponenten aufgetrennt wird. Anschließend werden beim Auslaufen des Zentrifugenrotors die einzelnen Blutkomponenten mit Hilfe von Druckluft oder Hydraulikdruck aus dem Blutbeutel verdrängt, so dass die Komponenten jeweils über eine Leitung in einen oder mehrere Satellitenbeutel überführt werden. Zur Durchführung dieses Arbeitsverfahrens ist jedoch eine spezielle Zentrifuge erforderlich, die einen Druckluftanschluss bzw. eine Hydraulikleitung für einen Aufnahmebehälter in der Zentrifuge bzw. einen Zentrifugenkorb bereitstellt, in den der Blutbeutel mit dem zu separierenden Blut eingebracht wird. Der Aufbau einer derartigen Zentrifuge erfordert, verglichen mit Zentrifugen, die ausschließlich zum Auftrennen des Bluts verwendet werden, einen höheren technischen Aufwand, was zu hohen Anschaffungskosten für diese Zentrifugen führt.

Ferner ist aus US 2006 / 0 122 048 A1 ein System zum zentrifugalen Auftrennen von in einem ersten Blutbeutel aufgenommen Blut in einzelne Blutkomponenten bekannt. Nach der Auftrennung des Bluts in die einzelnen Blutkomponenten wird eine Blutkomponente, z.B. Blutplasma, unter Verwendung einer Pumpenanordnung, z.B. einer Rollenpumpe, die in einem Rotor konzentrisch angeordnet ist, vom ersten Blutbeutel über eine Leitung, die durch die Pumpenanordnung verläuft, in einen zweiten Beutel gepumpt.

In einer Ausführungsform der US 2006 / 0 122 048 A1 kann außerdem eine weitere Auftrennung der im zweiten Blutbeutel aufgenommenen Blutkomponente durchgeführt werden. Anschließend wird eine durch diese weitere Auftrennung erhaltene Unterkomponente der Blutkomponente mittels eines sog. Expressors von dem zweiten Beutel in einen dritten Beutel überführt.

Weitere Blutseparationsvorrichtungen sind zudem aus US 2002 / 0 151 423 A1, WO 92 / 00145 A1, CN 108 578 801 A und US 2012 / 0 289 926 A1 bekannt. US 2012/289926 A1 offenbart eine Blutseparationsvorrichtung zum Separieren von Blut in mehrere Blutkomponenten unter Einfluss einer während eines Zentrifugiervorgangs in einer Zentrifuge erzeugten Zentrifugalkraft, mit: einer ersten Kammer zur Aufnahme eines Blutbeutels, der mit dem zu separierenden Blut gefüllt ist, das sich unter Einfluss der Zentrifugalkraft in die einzelnen Blutkomponenten derart auftrennt, dass sich jede Blutkomponente nach Beendigung einer ersten Phase des Zentrifugiervorgangs - in Richtung der Zentrifugalkraft gesehen - entsprechend ihrem spezifischen Gewicht in einem separaten Abschnitt des Blutbeutels befindet, einer zweiten Kammer zur Aufnahme mindestens eines Komponentenbeutels, der zur Befüllung mit einer jeweils zugeordneten Blutkomponente vorgesehen ist, wobei eine Einlassöffnung jedes Komponentenbeutels über eine Leitung, in der ein steuerbares Ventil angeordnet ist, mit einer Ausgangsöffnung des Blutbeutels verbunden ist, einem Verdrängungskörper, der in der ersten Kammer angeordnet ist und auf den Blutbeutel eine im Wesentlichen senkrecht zur Separationsrichtung gerichtete Presskraft ausübt, wobei der Verdrängungsköper so geformt ist, dass er die Presskraft mit Hilfe der Zentrifugalkraft erzeugt, und einer Steuereinheit, die während einer zweiten Phase des Zentrifugiervorgangs jedes Ventil so lange öffnet, bis die diesem Ventil zugeordnete Blutkomponente infolge der Presskraft des Verdrängungskörpers in den zugeordneten Komponentenbeutel gelangt ist.

Es ist Aufgabe der vorliegenden Erfindung eine kostengünstige und einfach zu verwendende Blutseparationsvorrichtung bereitzustellen. Die erfindungsgemäße Blutseparationsvorrichtung soll insbesondere für konventionelle Zentrifugen anwendbar sein, die keinen Druckluftanschluss bzw. Hydraulikanschluss aufweisen. Folglich können der technische Aufwand sowie die hohen Kosten für die Anschaffung einer neuen Zentrifuge vermieden werden.

Die vorliegende Erfindung löst die Aufgabe durch die Vorrichtung mit den Merkmalen des Anspruchs 1. Weitere vorteilhafte Ausgestaltungen der Erfindung sind in den abhängigen Ansprüchen angegeben.

Gemäß einem Aspekt der vorliegenden Erfindung ist die Blutseparationsvorrichtung so ausgebildet, dass die einzelnen Blutkomponenten durch einen Verdrängungskörper aus dem Blutbeutel verdrängt werden, der so geformt ist, dass er eine im Wesentlichen zur Zentrifugalkraft senkrechte Presskraft mit Hilfe einer während eines Zentrifugiervorgangs erzeugten Zentrifugalkraft und/oder mit Hilfe einer Federkraft erzeugt. Die einzelnen Blutkomponenten gelangen anschließend in entsprechende Komponentenbeutel, wobei eine Einlassöffnung jedes Komponentenbeutels über eine Leitung, in der ein steuerbares Ventil angeordnet ist, mit einer Ausgangsöffnung des Blutbeutels verbunden ist. Die erfindungsgemäße Blutseparationsvorrichtung weist darüber hinaus eine Steuereinheit auf, die nach Beendigung einer ersten Phase des Zentrifugiervorgangs, in der das Blut in die einzelnen Blutkomponenten aufgetrennt wird, jedes Ventil so lange öffnet, bis die diesem Ventil zugeordnete Blutkomponente infolge der Verdrängung mit Hilfe der durch den Verdrängungskörper erzeugten Presskraft in den zugeordneten Komponentenbeutel gelangt ist. Ein Überführen der einzelnen Blutkomponenten in die entsprechenden Komponentenbeutel wird folglich während einer zweiten Phase des Zentrifugiervorgangs durchgeführt. Die Zentrifugalkraft während der zweiten Phase ist dabei kleiner als die Zentrifugalkraft während der ersten Phase. Die zweite Phase ist dabei bevorzugt eine Phase, bei der die Zentrifuge so eingestellt wird, dass eine vorgegebene Zentrifugalkraft wirkt. Alternativ kann die zweite Phase ein Auslaufen eines Zentrifugenrotors sein.

Es ist anzumerken, dass eine Presskraft, die mit Hilfe der Federkraft erzeugt wird, bereits beim Einbringen des Blutbeutels in die Blutseparationsvorrichtung auf den Blutbeutel drückt. Durch ein von einer Steuereinheit gesteuertes Öffnen und Schließen der Ventile in den Leitungen vom Blutbeutel zu den Komponentenbeuteln kann jedoch das Fließen der einzelnen Blutkomponenten zu den entsprechenden Komponentenbeuteln gezielt beeinflusst werden, so dass es nicht erforderlich ist, die Presskraft, wie in DE 29 38 367 A1 beschrieben, nach dem Auftrennen des Bluts in die einzelnen Blutkomponenten anzulegen.

In der folgenden Beschreibung werden Modifikationen der erfindungsgemäßen Blutseparationsvorrichtung sowie unterschiedliche Ausführungsformen zum Separieren der einzelnen Blutkomponenten unter Bezugnahme auf die beigefügten Figuren beschrieben. Es zeigen:
Fig. 1 eine Modifikation einer Blutseparationsvorrichtung;
Fig. 2 die Modifikation der Blutseparationsvorrichtung, in die eine Steuereinheit sowie ein Blutbeutel und zwei Komponentenbeutel eingebracht sind, bei Verwendung gemäß einer Ausführungsform;
Fig. 3 die Modifikation der Blutseparationsvorrichtung bei Verwendung gemäß der Ausführungsform nach Beendigung einer ersten Phase eines Zentrifugiervorgangs, bei dem Blut in einzelne Komponenten aufgetrennt wird;
Fig. 4 die Modifikation der Blutseparationsvorrichtung bei Verwendung gemäß der Ausführungsform, bei der eine der Blutkomponenten vom Blutbeutel über eine Leitung in einen der Komponentenbeutel gelangt ist;
Fig. 5 die Modifikation der Blutseparationsvorrichtung bei Verwendung gemäß der Ausführungsform, bei der eine andere Blutkomponente vom Blutbeutel über eine andere Leitung in einen anderen Komponentenbeutel gelangt ist;
Fig. 6 eine weitere Modifikation einer Blutseparationsvorrichtung;
Fig. 7 die weitere Modifikation der Blutseparationsvorrichtung, in die die Steuereinheit sowie der Blutbeutel und die zwei Komponentenbeutel eingebracht sind, bei Verwendung gemäß der Ausführungsform;
Fig. 8 die weitere Modifikation der Blutseparationsvorrichtung bei Verwendung gemäß der Ausführungsform nach Beendigung der ersten Phase des Zentrifugiervorgangs, beim dem das Blut in die einzelnen Komponenten aufgetrennt wird;
Fig. 9 die weitere Modifikation der Blutseparationsvorrichtung bei Verwendung gemäß der Ausführungsform, bei der eine der Blutkomponenten vom Blutbeutel über eine Leitung in einen der Komponentenbeutel gelangt ist;
Fig. 10 die weitere Modifikation der Blutseparationsvorrichtung bei Verwendung gemäß der Ausführungsform, bei der eine andere Blutkomponente vom Blutbeutel über eine andere Leitung in einen anderen Komponentenbeutel gelangt ist;
Fig. 11 eine der Modifikationen der Blutseparationsvorrichtung bei Verwendung gemäß einer weiteren Ausführungsform, bei der abwechselnd eine erste Klemme und eine zweite Klemme geöffnet werden, so dass eine Blutkomponente bzw. eine andere Blutkomponente über entsprechende Leitungen in einen jeweiligen Komponentenbeutel gelangen;
Fig. 12A und Fig. 12B eine weitere Modifikation der Blutseparationsvorrichtung, bei der ein Verdrängungskörper, der eine Hebelanordnung mit einer kardanischen Lagerung und einer Pressplatte aufweist, verwendet wird;
Fig. 13A und 13B eine Front- bzw. eine Seitenansicht einer Führungsplatte zum Führen von Leitungen zwischen dem Blutbeutel und den Komponentenbeuteln; und
Fig. 14 eine Seitenansicht einer Blutseparationsvorrichtung, an der die Führungsplatte angebracht ist.

Die erfindungsmäße Blutseparationsvorrichtung 4 kann unter Verwendung von unterschiedlichen Modifikationen verwirklicht werden. Aus diesem Grund werden zunächst die unterschiedlichen Modifikationen beschrieben. In einem späteren Abschnitt der Beschreibung werden die unterschiedlichen Ausführungsformen zur Verwendung der erfindungsgemäßen Blutseparationsvorrichtung 4 erläutert. Es ist anzumerken, dass die unterschiedlichen Ausführungsformen mit allen Modifikationen der Blutseparationsvorrichtung 4 ausgeführt werden können.

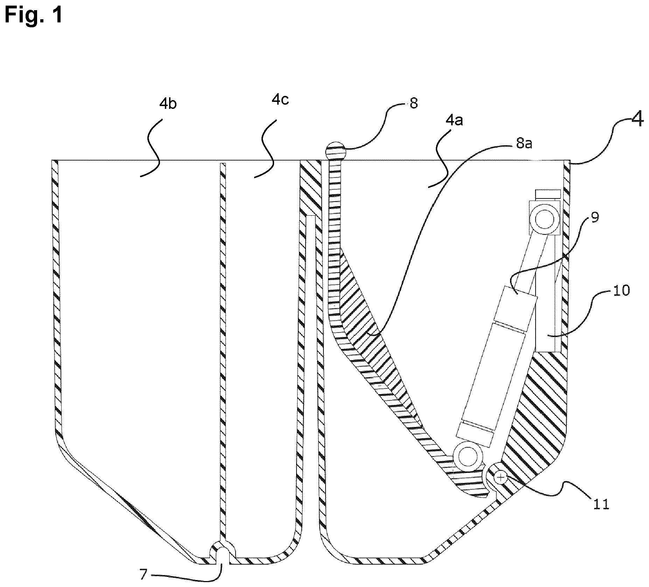

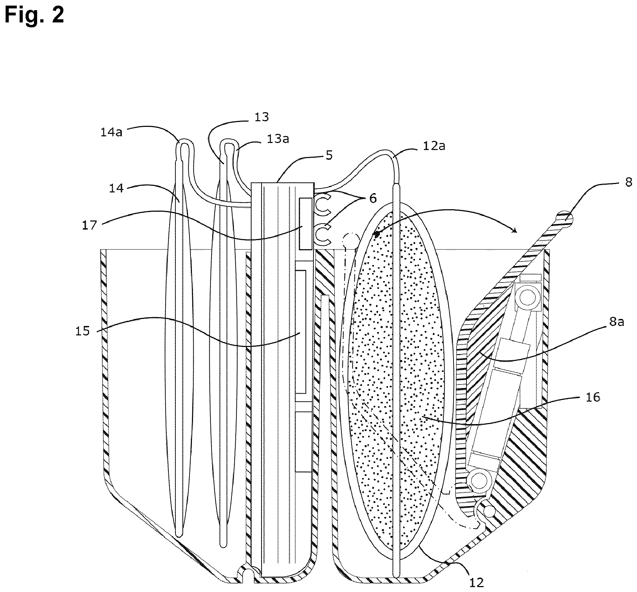

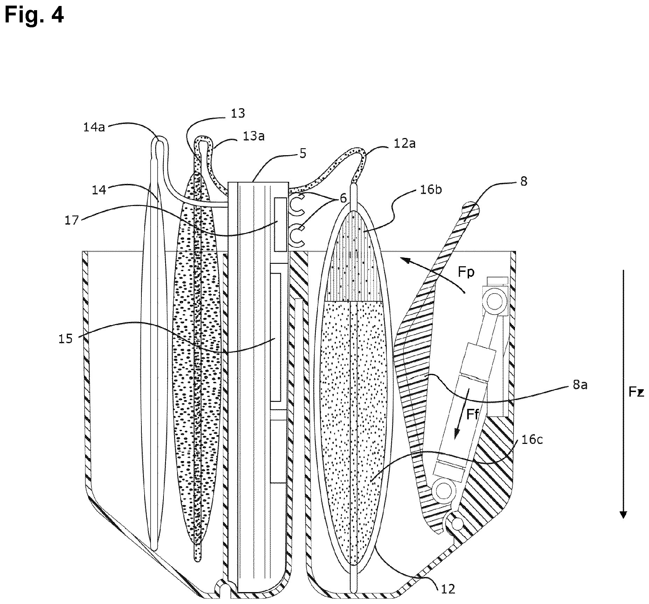

Fig. 1 und Fig. 2 zeigen den Aufbau einer Blutseparationsvorrichtung 4 gemäß einer Modifikation. Die Blutseparationsvorrichtung 4 weist eine erste Kammer 4a, eine zweite Kammer 4b und eine Kammer 4c für eine Steuereinheit 5 auf. In der ersten Kammer 4a ist ein Verdrängungskörper 8 angeordnet, der eine Hebelanordnung aufweist, die an einer Wandung der ersten Kammer 4a an einer Rotationsachse 11 drehbar angebracht ist. Die Wandung ist dabei, wie in den Figuren zu sehen, eine Wandung der ersten Kammer die zur Außenseite der Blutseparationsvorrichtung 4 gerichtet ist. Der Verdrängungskörper 8 ist dafür vorgesehen, dass er, wie z.B. in Fig. 4 gezeigt, eine Presskraft Fp erzeugt, um die einzelnen Blutkomponenten 16a, 16b und 16c aus dem Blutbeutel 12 zu drücken, so dass diese in entsprechende Komponentenbeutel 13 bzw. 14 gelangen.

In dieser Modifikation ist der Verdrängungskörper 8 so geformt, dass er die Presskraft Fp mit Hilfe einer beim Zentrifugieren erzeugten Zentrifugalkraft Fz und mit Hilfe einer Federkraft Ff erzeugt.

Zum Erzeugen der Presskraft Fp mit Hilfe der Zentrifugalkraft Fz weist die Hebelanordnung des Verdrängungskörpers 8 ein Gewicht 8a auf, dessen Schwerpunkt, wie in Fig. 2 zu sehen, zu einer vertikal durch die Rotationsachse 11 verlaufenden y-Achsenrichtung in Richtung der zweiten Kammer 4b, d.h. in den Figuren nach links, versetzt angeordnet ist. Folglich verursacht die Zentrifugalkraft Fz ein Drehmoment an der Hebelanordnung des Verdrängungskörpers 8 entgegen dem Uhrzeigersinn, wodurch die Hebelanordnung des Verdrängungskörpers 8 in Richtung der zweiten Kammer 4b gedrückt wird. Ein oberer Abschnitt der Hebelanordnung, der in einem vorgegebenen Winkel, wie z.B. 45°, zum unteren Abschnitt der Hebelanordnung in Richtung einer Außenseite der Blutseparationsrichtung abgewinkelt ist, wird aufgrund der Drehung in Richtung der gegenüberliegenden Wandung gedrückt und ist im Wesentlichen parallel zu einer innenliegenden Wandung der ersten Kammer 4a. Der obere Abschnitt verteilt daher die Presskraft Fp, die durch die Zentrifugalkraft Fz erzeugt wird, auf eine größere Fläche der innenliegenden Wandung der ersten Kammer 4a. Zudem stellt diese abgewinkelte Auslegung des Hebelarms den Vorteil bereit, dass der untere Abschnitt des Hebelarms weiter nach links gedreht werden kann, wodurch das anliegende Drehmoment vergrößert wird, da das Gewicht 8a des Verdrängungskörpers 8 zur vertikal durch die Rotationsachse 11 verlaufenden y-Achsenrichtung weiter nach links verschoben ist.

Zusätzlich weist die Blutseparationsvorrichtung 4 gemäß dieser Modifikation eine Feder 9 auf, deren eines Ende an der Wandung der ersten Kammer 4a und deren anderes Ende am Verdrängungskörper 8 angebracht ist, so dass eine Federkraft Ff der Feder eine Presskraft Fp erzeugt, die im Wesentlichen zur der mit Hilfe der Zentrifugalkraft Fz erzeugten Presskraft Fp gleichgerichtet ist. Die Federkraft der Feder 9 kann bevorzugt mit Hilfe einer Führungsschiene 10 dadurch eingestellt werden, dass der Federweg durch Verschieben des daran angebrachten Endes der Feder 9 verändert wird. Wird das angebrachte Ende der Feder 9 nach oben verschoben, wird der Federweg verkürzt und die Federkraft Ff nimmt ab. Wird das angebrachte Ende der Feder 9 nach unten verschoben, wird der Federweg verlängert und die Federkraft Ff nimmt zu. Die resultierende Presskraft Fp, die auf den Blutbeutel 12 drückt, ist folglich die Summe aus der mit Hilfe der Zentrifugalkraft Fz erzeugten Presskraft Fp und der mit Hilfe der Federkraft Ff erzeugten Presskraft Fp. In einem Zustand, in dem kein Blutbeutel 12 in der ersten Kammer 4a aufgenommen ist, wird der Verdrängungskörper 8 somit, wie in Fig. 1 sowie in Fig. 2 gestrichelt dargestellt, an die innenliegende Wandung der ersten Kammer 4a gedrückt.

Die Blutseparationsvorrichtung 4 gemäß einer anderen Modifikation (in den Figuren nicht gezeigt) weist kein Gewicht 8a an der Hebelanordnung des Verdrängungskörpers 8 auf. Folglich wird die Presskraft Fp fast ausschließlich durch die Federkraft Ff der Feder 9 erzeugt. Lediglich die Zentrifugalkraft Fz, die an der Masse der Hebelanordnung selbst angreift, erzeugt eine vernachlässigbare Presskraft Fp. Die Federkraft Ff ist dabei durch Auslegung der Feder selbst oder durch Verschieben des einen Endes an der Führungsschiene 10 so auszulegen, dass die durch die Federkraft Ff erzeugte Presskraft Fp, die folglich der gesamten erzeugten Presskraft Fp entspricht, ausreicht, dass die einzelnen Blutkomponenten 16a, 16b und 16c aus dem Blutbeutel 12 gedrückt werden und über die entsprechenden Leitungen 13a, 14a in die dafür vorgesehenen Komponentenbeutel 13 und 14 gelangen. Bei dieser Modifikation wird der Verdrängungskörper 8 aufgrund der Federkraft Ff der Feder 9 ebenfalls, ähnlich wie in Fig. 1, an die innenliegenden Wandung der ersten Kammer 4a gedrückt.

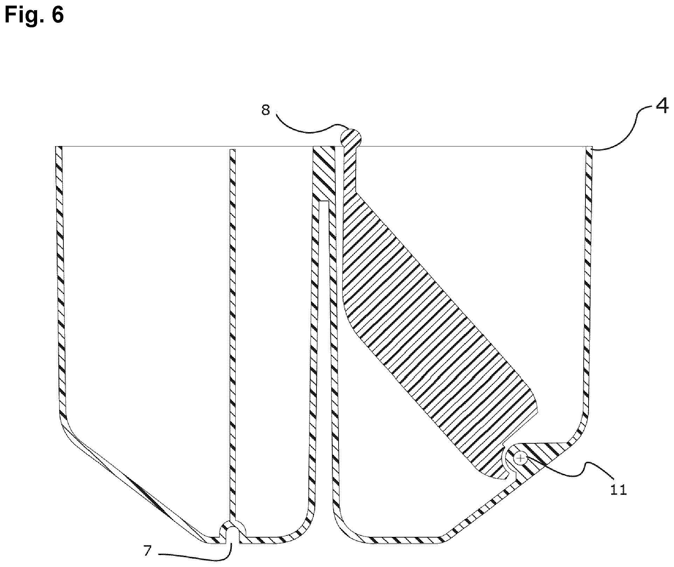

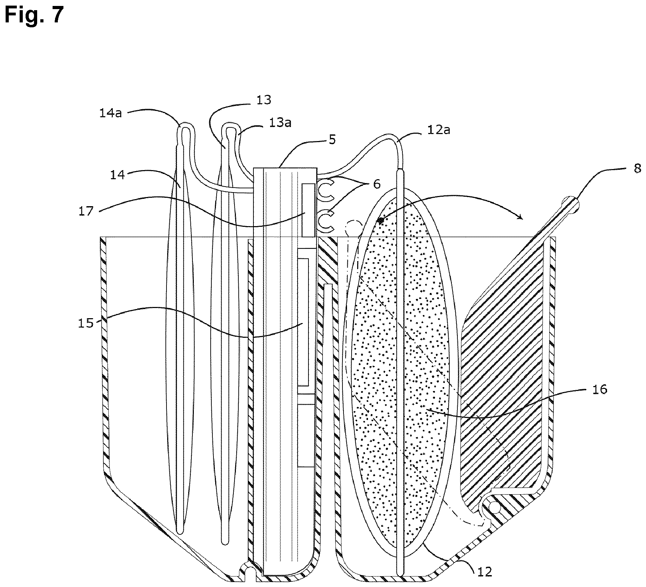

Die Blutseparationsvorrichtung 4 gemäß einer weiteren Modifikation ist in Fig. 6 gezeigt und erzeugt die erforderliche Presskraft Fp ausschließlich mit Hilfe der beim Zentrifugieren erzeugten Zentrifugalkraft Fz. Aus diesem Grund weist der Verdrängungskörper 8 gemäß dieser Modifikation eine größere Masse als der Verdrängungskörper 8 gemäß den oben beschriebenen Modifikationen auf, so dass die mit Hilfe der Zentrifugalkraft Fz erzeugte Presskraft Fp, die folglich der gesamten erzeugen Presskraft Fp entspricht, ausreicht, um die einzelnen Blutkomponenten 16a, 16b und 16c aus dem Blutbeutel 12 zu drücken. Die Masse der Hebelanordnung ist dabei so am Verdrängungskörper 8 verteilt, dass der Schwerpunkt des Verdrängungskörpers zu einer vertikal durch die Rotationsachse 11 verlaufenden y-Achsenrichtung nach links verschoben ist. Dadurch ist der Verdrängungskörper 8 in einem Ausgangszustand, wie in Fig. 6 und in Fig. 7 gestrichelt gezeigt, in Richtung der innenliegenden Wandung der ersten Kammer 4a ausgelenkt. Durch diese Gewichtsverteilung und dem daraus resultierenden nach links verschobenen Schwerpunkt wird sichergestellt, dass unter Einfluss der erzeugten Zentrifugalkraft Fz ein Drehmoment an der Hebelanordnung des Verdrängungskörpers 8 angreift, das entgegen dem Uhrzeigersinn gerichtet ist. Folglich wird die Presskraft Fp erzeugt, die in Richtung der innenliegenden Wandung der ersten Kammer 4a drückt. Wie bei den vorhergehend beschriebenen Modifikationen ist ein oberer Abschnitt der Hebelanordnung des Verdrängungskörpers 8 in Richtung zur Außenseite der Blutseparationsrichtung in einem vorgegebenen Winkel abgewinkelt. Auf diese Weise wird die Presskraft Fp ebenfalls auf einer größeren Fläche verteilt und ein unterer Abschnitt des Verdrängungskörpers 8 kann weiter nach links ausgelenkt werden.

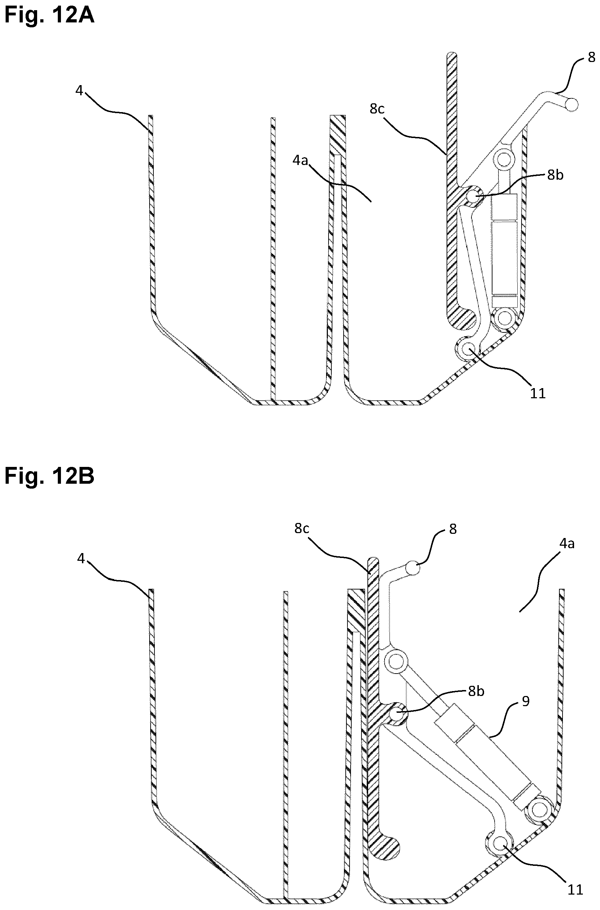

Gemäß einer anderen Modifikation, die in den Figuren 12A und 12B gezeigt ist, kann der Verdrängungskörper 8 eine Hebelanordnung, an der eine kardanischen Lagerung 8b und einer Pressplatte 8c angebracht sind, aufweisen. Durch die kardanische Lagerung 8b wird sichergestellt, dass die Pressplatte 8c zu der innenliegenden Wandung der ersten Kammer 4a parallel angeordnet ist. Durch Verwendung der Hebelanordnung, an der die kardanische Lagerung 8b und die Pressplatte 8b angebracht sind, wird eine Fläche, in der der Verdrängungskörper 8 auf den Blutbeutel 12 drückt, vergrößert, so dass die einzelnen Blutkomponenten 16a, 16b und 16c noch zuverlässiger aus dem Blutbeutel 12 gedrückt werden.

Die Blutseparationsvorrichtung 4 gemäß den beschriebenen Modifikationen weist somit den Verdrängungskörper 8 auf, der eine Presskraft Fp in Richtung der innenliegenden Wandung der ersten Kammer 4a mit Hilfe der während eines Zentrifugiervorgangs erzeugten Zentrifugalkraft Fz und/oder mit Hilfe der durch die Feder 9 bereitgestellten Federkraft Ff erzeugt. Der Blutbeutel 12 wird somit, wie in Fig. 2 gezeigt, in einem eingebrachten Zustand, durch den Verdrängungskörper 8 an die innenliegende Wandung der ersten Kammer 4a gedrückt, so dass die einzelnen Blutkomponenten 16a, 16b und 16c aus dem Blutbeutel 12 gedrückt werden.

Demzufolge benötigt die erfindungsgemäße Blutseparationsvorrichtung 4 zum Erzeugen der Presskraft Fp keinen zusätzlichen Druckluft- oder Hydraulikanschluss. Es ist daher möglich, die erfindungsgemäße Blutseparationsvorrichtung 4 als einen offenen Behälter, nachfolgend als Zentrifugeninlet oder Inlet bezeichnet, auszubilden, dessen Außengestalt an die Innenform eines Aufnahmebehälters einer konventionellen Zentrifuge anpassbar ist. Das Inlet kann darüber hinaus so ausgebildet sein, dass es direkt an einen Rotor einer Zentrifuge anbringbar ist, so dass kein Aufnahmebehälter erforderlich ist. Die erfindungsgemäße Blutseparationsvorrichtung 4 kann daher für bereits vorhandene Zentrifugen ohne Druckluft- oder Hydraulikanschluss verwendet werden, wodurch eine kostenaufwändige Anschaffung einer speziellen Zentrifuge, die einen hohen technischen Aufwand erfordert, vermieden wird.

Im Folgenden wird die Verwendung der Blutseparationsvorrichtung 4 gemäß unterschiedlichen Ausführungsformen beschrieben. Es ist anzumerken, dass die in den Aufnahmebehälter eingebrachte Blutseparationsvorrichtung 4 aufgrund der erzeugten Zentrifugalkraft Fz um 90° gegen den Uhrzeigersinn ausgelenkt wird, da der Aufnahmebehälter einer Zentrifuge für gewöhnlich am oberen Ende in der Mitte an einem Rotor der Zentrifuge drehbar angebracht ist. Demzufolge befindet sich die erste Kammer 4a während des Zentrifugierens oberhalb der zweiten Kammer 4b. Die Hebelanordnung des Verdrängungskörpers 8 wird somit beim Zentrifugieren nach unten gedrückt. Diese Anordnung bietet den zusätzlichen Vorteil, dass die Gewichtskraft der Hebelanordnung in Verbindung mit dem verschobenen Schwerpunkt der Hebelanordnung sicherstellt, dass der Verdrängungskörper 8 die Presskraft Fp in Richtung der innenliegenden Wandung der ersten Kammer 4a drückt. Zum Zweck einer einfacheren Beschreibung werden die Richtungen im Folgenden jedoch wie in den Figuren angegeben.

In der ersten Ausführungsform sind der Blutbeutel 12 und die Komponentenbeutel 13 und 14 gemäß einer sog. Top-Top-Spezifikation angeordnet. Das bedeutet, dass die Leitungen zu den beiden Komponentenbeuteln 13 und 14 am selben Ende, z.B. an einem oberen Ende, des Blutbeutels 12 angeschlossen sind. Der Blutbeutel 12 weist eine Öffnung auf, an der eine Leitung 12a angeschlossen ist, die sich an einer Y-Abzweigung in zwei Leitungen 13a und 14a aufteilt, die mit einem entsprechenden Komponentenbeutel 13 bzw. 14 verbunden sind. In der Leitung 12a ist ein Brechventil angeordnet, so dass der Blutbeutel 12 in einem Ausgangszustand sicher verschlossen ist. Die Leukozyten des Bluts 16, das im Blutbeutel 12 aufgenommen ist, können vorab mit Hilfe eines Leukozytenfilters entfernt werden.

In den Figuren 2 und 7 wird der Ausgangszustand gezeigt, bevor die Blutseparationsvorrichtung 4 in die Zentrifuge eingebracht wird. Der mit dem zu separierenden Blut 16 gefüllte Blutbeutel 12 mit dem intakten Brechventil wird in die erste Kammer 4a eingebracht, indem der Verdrängungskörper 8 per Hand nach rechts in Richtung der außenliegenden Wandung gedrückt wird, an der der Verdrängungskörper 8 drehbar angebracht ist. In den Figuren 2 und 7 ist die Ausgangslage des Verdrängungskörpers 8 durch eine strichpunktierte Linie gezeigt, und die Handlung des Nach-Hinten-Drückens wird durch einen gebogenen durchgezogenen Pfeil beschrieben. Nachdem der Verdrängungskörper 8 losgelassen wird, drückt dieser auf den Blutbeutel 12, wobei die Presskraft Fp bei Modifikationen mit einer Federkraft Ff im Ausganszustand höher als bei Modifikationen ohne Federkraft Ff ist. Durch das intakte Brechventil wird jedoch verhindert, dass Blut 16, das noch nicht in die einzelnen Blutkomponenten aufgetrennt ist, aus dem Blutbeutel 12 gedrückt wird und in die Komponentenbeutel 13 und 14 gelangt.

Eine Steuereinheit 5 wird in die Kammer 4c der Blutseparationsvorrichtung 4 eingebracht und ist so ausgelegt, dass sie eine Verteilung der Blutkomponenten 16a, 16b und 16c auf die Komponentenbeutel 13 und 14 steuert, indem sie Ventile in den einzelnen Leitungen 12a, 13a und 14a entsprechend öffnet und schließt. Die Steuereinheit 5 weist aus diesem Grund einen Mikrocomputer oder eine andere logische Einheit auf, der einen Speicher aufweist, in dem ein entsprechendes Softwareprogramm gespeichert ist, das die Verteilung der Blutkomponenten 16a, 16b, 16c steuert. Die Ventile sind bei den vorliegenden Modifikationen durch Klemmen ausgebildet, die jeweils mit Hilfe eines Elektromotors 15 angetrieben werden, die wiederum von dem Mikrocomputer der Steuereinheit 5 angesteuert werden. Die Klemmen sowie die dazugehörigen Elektromotoren 15 sind ebenfalls an der Steuereinheit 5 angebracht und die Steuereinheit 5 wird vorteilhafterweise mit Hilfe einer unabhängigen Energieversorgung, wie etwa Batterien, betrieben, so dass keine äußeren Anschlüsse erforderlich sind, wodurch der technische Aufwand der erfindungsgemäßen Blutseparationsvorrichtung 4 weiter reduziert wird. Aufgrund des geringen Energiebedarfs des Elektromotors und des Mikrocomputers reicht eine Ladung der Batterien für mehrere Separationsvorgänge.

Die Leitungen 12a, 13a und 14a werden wie folgt in die Klemmen eingebracht und am Inlet angeordnet. Die Leitung 12a, die an der Öffnung des Blutbeutels 12 angeschlossen ist, wird durch eine erste Klemme geführt, die in einem oberen Abschnitt der Steuereinheit 5 angeordnet ist, der aus dem Inlet hervorragt. Die erste Klemme wird anschließend verschlossen, da das Brechventil zu öffnen ist, wenn das Inlet in die Zentrifuge eingebracht wird. Auf diese Weise wird auch nach dem Öffnen des Brechventils in der Leitung 12a verhindert, dass nicht separiertes Blut 16 durch die bereits anliegende Presskraft Fp in einen der Komponentenbeutel 13 und 14 gelangt. Es ist dazu anzumerken, dass alle Klemmen, in die die Leitungen 12a, 13a, 14a jeweils eingebracht werden sollen, so anzuordnen und auszubilden sind, dass ein Zusammendrücken und/oder Knicken der darin aufgenommenen Leitungen 12a, 13a, 14a im Falle einer geöffneten Klemme während des Zentrifugiervorgangs vermieden wird, so dass ein Fließweg in den Leitungen 12a, 13a, 14a zuverlässig sichergestellt ist.

Anschließend wird die Leitung 12a an der Außenseite des Inlets, die sich in den Figuren näher am Betrachter befindet, entlang der Wandung zwischen der Kammer 4c für die Steuereinheit 5 und der zweiten Kammer 4b nach unten verlegt und anschließend an der äußeren Bodenfläche des Inlets auf die andere Seite des Inlets (nach hinten in den Figuren) geführt. Damit die Leitung beim Einbringen in den Aufnahmebehälter der Zentrifuge nicht abgedrückt oder geknickt wird, ist eine Nut 7 in die äußere Fläche des Inlets eingebracht. Die Y-Abzweigung befindet sich bei dieser Verlegung der Leitung im Bereich der Mitte der äußeren Bodenfläche und ist in eine dafür vorgesehene Vertiefung einzubringen. Von dort aus verlaufen zwei Nuten 7 an der Außenseite des Inlets, um die Leitungen 13a und 14a jeweils einzeln in einer dafür bereitgestellte Nut 7 aufzunehmen. In einem oberen Abschnitt des Inlets sind zwei Halterungen 6 an der Steuereinheit angeordnet, in die die Leitungen 13a und 14a einfügbar sind. Auf diese Weise sind die Leitungen 12a, 13a und 14a am Inlet befestigt und die umlaufenden Nuten 7 sowie die Vertiefung für die Y-Abzweigung ermöglichen es, dass das Inlet in den Aufnahmebehälter eingebracht werden kann, ohne dass eine der Leitungen 12a, 13a, 14a abgedrückt oder die Y-Abzweigung zerstört wird.

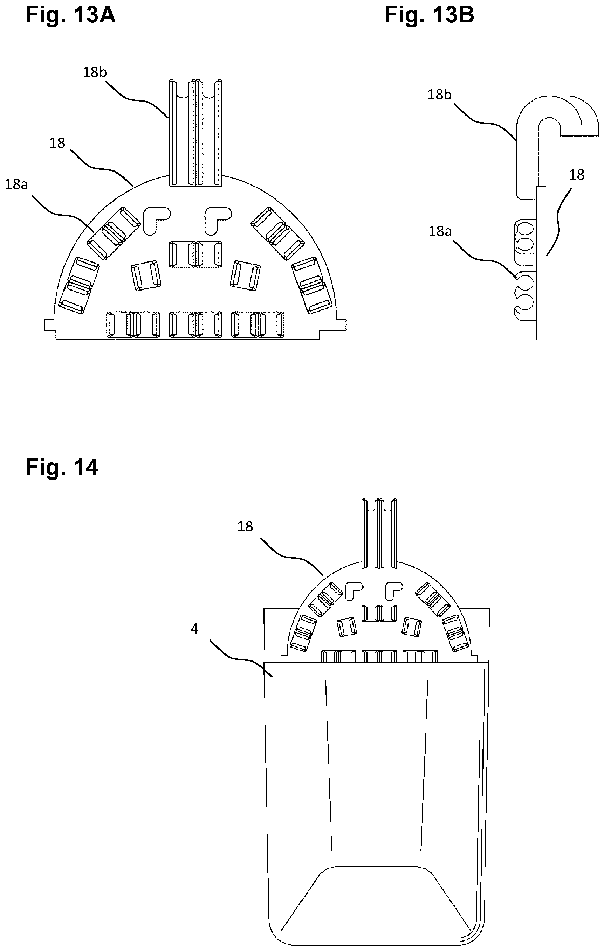

Alternative oder zusätzlich zu einer Führung der Leitungen 12a, 13a, 14a an der Blutseparationsvorrichtung 4 selbst, kann eine in den Figuren 13A und 13B gezeigte Führungsplatte 18 zum Führen der Leitungen 12a, 13a, 14a verwendet werden. Die Leitungen 12a, 13a, 14a werden dazu in die einzelnen Clips 18a der Führungsplatte 18 eingefügt, so dass verhindert wird, dass die Leitungen während eines Zentrifugiervorgangs abgeknickt oder zusammengedrückt werden. Die Führungsplatte 18 weist zudem zwei Schienen 18b auf, die die Leitung 13a und 14a zur zweiten Kammer 4b, in der die Komponentenbeutel 13 und 14 aufgenommen sind, führen. In Fig. 14 ist eine Blutseparationsvorrichtung 4 gezeigt, an der die Führungsplatte 18 angebracht ist. Die Führungsplatte 18 wird dabei an einem oberen Abschnitt der innenliegenden Wandung der ersten Kammer 4a durch eine Klemme, eine Schraube oder eine andere geeignete Halteeinheit angebracht. Die Führungsplatte 18 ist nicht auf die gezeigte Ausgestaltung bzw. Anordnung der Clips 18a begrenzt und kann an diverse Blutbeutelsysteme mit unterschiedlichen Leitungslängen und Leitungsdurchmessern angepasst werden, um ein Abknicken und/oder Zusammendrücken der Leitungen 12a, 13a, 14a zuverlässig zu verhindern, so dass ein Fließweg in den Leitungen 12a, 13a, 14a zuverlässig sichergestellt ist.

Die Leitungen 13a und 14a, die mit den Komponentenbeuteln 13 bzw. 14 verbunden sind, werden durch eine zweite Klemme geführt, wobei die zweite Klemme so ausgebildet ist, dass sie beide Leitungen aufnimmt. Die zweite Klemme ist darüber hinaus so ausgebildet, dass sie nur eine der beiden Leitungen 13a und 14a öffnet, während die jeweils andere Leitung 13a und 14a unterbrochen wird. Folglich kann ein Transport einer Blutkomponente 16a, 16b, 16c nur in einen der beiden Komponentenbeutel 13 und 14 erfolgen. Es ist jedoch nicht zwingend erforderlich, dass diese Art für die Klemme verwendet wird und es können beispielsweise auch zwei einzelne Klemmen verwendet werden, wenn sichergestellt ist, dass zur selben Zeit nur eine der beiden Klemmen geöffnet ist.

Anschließend werden die Komponentenbeutel 13 und 14 in der zweiten Kammer 4b angeordnet, das Brechventil des Blutbeutels 12 geöffnet und das Inlet in einen Aufnahmebehälter einer Zentrifuge formschlüssig eingesetzt. Das Öffnen des Brechventils kann dabei auch erst nach Einsetzen des Inlets in den Aufnahmebehälter der Zentrifuge erfolgen. Durch die Verlegung der Leitungen 12a, 13a und 14a in den Nuten 7 an der Außenseite des Inlets bzw. in der Führungsplatte 18 wird beim Einbringen in den Aufnahmebehälter sichergestellt, dass die einzelnen Leitungen 12a, 13a und 14a nicht abgedrückt oder beschädigt werden. Zudem wird durch die Verlegung der Leitungen 12a, 13a und 14a um das Inlet herum oder in der Führungsplatte 18 verhindert, dass die einzelnen Leitungen 12a, 13a und 14a aus der Öffnung des Inlets hervorragen, so dass ein Risiko eines Verhakens mit anderen Teilen der Zentrifuge verringert bzw. vermieden wird.

Es ist anzumerken, dass der Verdrängungskörper 8, wie oben beschrieben, bereits auf den Blutbeutel 12 drückt. Es wird jedoch auch nach dem Aufbrechen des Brechventils durch die geschlossene erste Klemme sichergestellt, dass kein Blut 16 in einen der Komponentenbeutel 13 und 14 gelangt. Die zweite Klemme ist beim Einbringen in die Zentrifuge so konfiguriert, dass die Leitung 13a zum Komponentenbeutel 13 geöffnet und die Leitung 14a zum anderen Komponentenbeutel 14 unterbrochen ist.

Sind ein oder mehrere Inlets in die dafür vorgesehenen Aufnahmebehälter am Zentrifugenrotor eingebracht, wird die Zentrifuge gestartet. Zum Auftrennen von Blut 16 ist eine Zentrifugalkraft Fz im Bereich von 1500 g bis 3000 g erforderlich, wozu bei typischen Zentrifugen, die für Blutbeutel 12 vorgesehen sind, eine Drehzahl im Bereich von 3000 Umdrehungen pro Minute einzustellen ist. Wie weiter oben beschrieben, wird die Blutseparationsvorrichtung 4 etwa um 90° gegen den Uhrzeigersinn ausgelenkt. Die Zentrifugalkraft Fz wirkt folglich, wie in den Figuren dargestellt, von oben nach unten. Nach 5 bis 15 Minuten kann diese erste Phase des Zentrifugiervorgangs, bei der das Blut in die einzelnen Komponenten auftrennt wird, beendet werden.

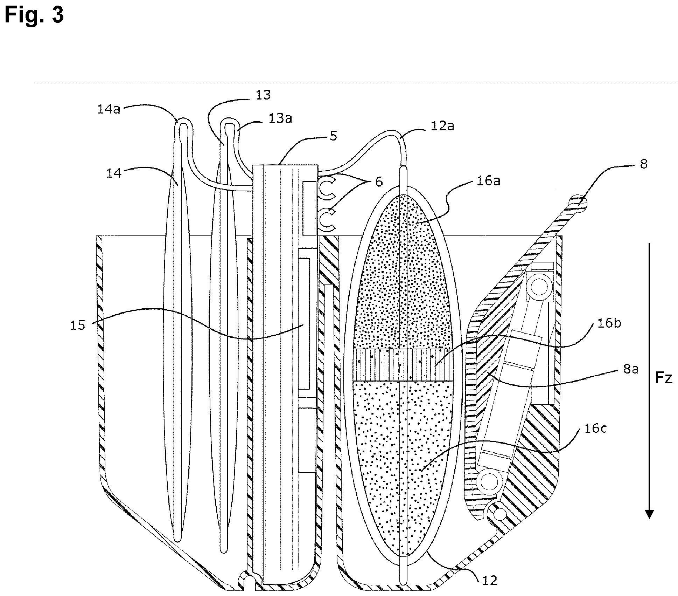

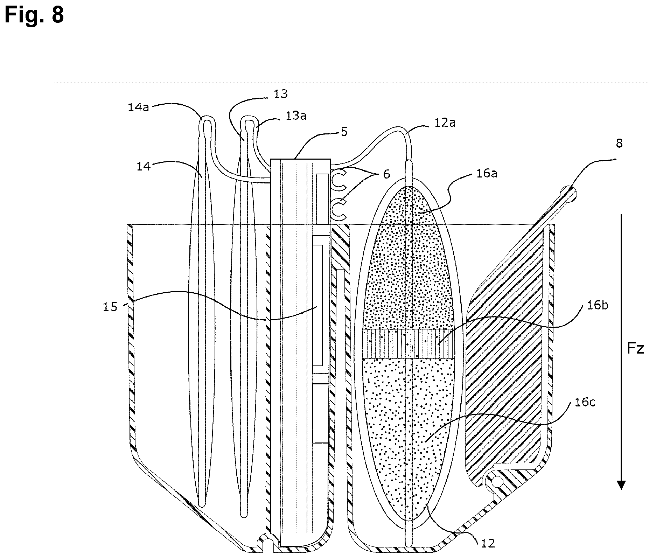

Während der ersten Phase des Zentrifugiervorgangs wird das Blut 16, wie in den Figuren 3 und 8 gezeigt, in die einzelnen Blutkomponenten 16a, 16b und 16cüberführt. Die einzelnen Blutkomponenten 16a, 16b und 16c sind entsprechend ihrem spezifischen Gewicht in Richtung der Zentrifugalkraft Fz angeordnet, wobei Blutkomponenten 16a, 16b und 16c mit einem höheren spezifischen Gewicht weiter außen, in den Figuren weiter unten, angeordnet sind. Die Richtung, in der die einzelnen Blutkomponenten 16a, 16b und 16c angeordnet sind, wird auch als Separationsrichtung bezeichnet. Die einzelnen Blutbestandteile in der vorliegenden Ausführungsform sind insbesondere Blutplasma 16a, Buffy-Coat 16b und Erythrozyten 16c.

Die erfindungsgemäße Blutseparationsvorrichtung 4 weist bevorzugt einen Lage-Sensor auf, der so ausgebildet ist, dass er die Lage des Buffy-Coat 16c im Blutbeutel 12 bestimmt. Es ist somit möglich den Hämatokrit des separierten Bluts 16 zu bestimmen. Der Lage-Sensor ist beispielsweise als optischer Sensor ausgebildet und wird zudem für eine weiter unten beschriebene Ausführungsform verwendet.

Nach Beendigung der ersten Phase des Zentrifugiervorgangs wird die Zentrifuge in einer zweiten Phase so eingestellt, dass eine geringere Zentrifugalkraft Fz als während der ersten Phase wirkt. Die zweite Phase ist dabei bevorzugt eine Phase, bei der die Zentrifuge so eingestellt wird, dass eine vorgegebene Zentrifugalkraft Fz wirkt. Es ist anzumerken, dass die Zentrifugalkraft Fz während der zweiten Phase kleiner als die Zentrifugalkraft Fz während der ersten Phase ist. Alternativ kann die zweite Phase ein Auslaufen eines Zentrifugenrotors sein.

Während der zweiten Phase werden die Blutkomponenten 16a, 16b und 16c in die entsprechenden Komponentenbeutel 13 und 14 überführt, so dass sie für Untersuchungen und/oder therapeutische Maßnahmen zur Verfügung stehen. Aus diesem Grund wird als erstes die erste Klemme geöffnet und der Verdrängungskörper 8, der bereits mit der Presskraft Fp auf den Blutbeutel 12 drückt, drückt die Blutkomponenten 16a, 16b und 16c in Richtung der Separationsrichtung aus dem Blutbeutel 12. Ein Überführen der Blutkomponenten 16a, 16b und 16c während der zweiten Phase, bietet darüber hinaus den Vorteil, dass ein erneutes Vermischen der einzelnen Blutkomponenten 16a, 16b und 16c vermieden wird, wenn der Blutbeutel 12 für längere Zeit wieder in einer vertikalen Ausrichtung gelagert wird, wie es beispielsweise bei Verwendung einer separaten Blutseparationsvorrichtung erforderlich ist.

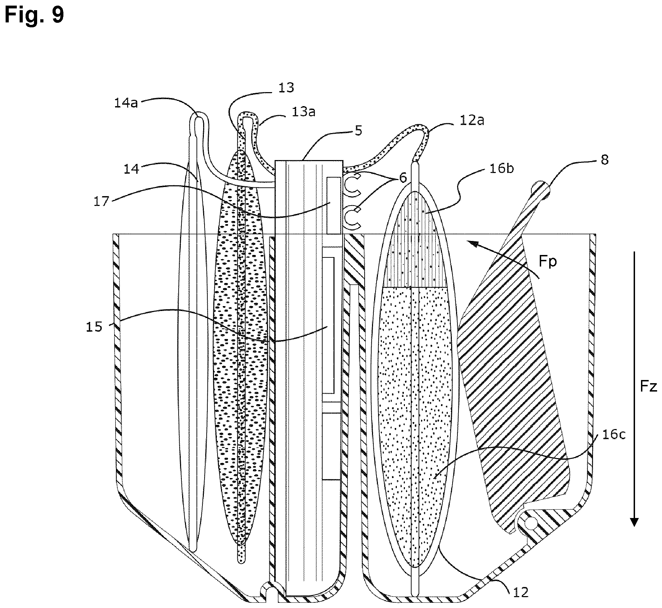

Demzufolge wird zuerst das Plasma 16a, wie in den Figuren 4 und 9 gezeigt, aus dem Blutbeutel 12 gedrückt und gelangt über die Leitungen 12a und 13a in den für Plasma vorgesehenen Komponentenbeutel 13.

Die Steuereinheit weist einen Sensor 17 auf, der ermittelt, ob eine einer Klemme zugeordnete Blutkomponente vollständig aus dem Blutbeutel 12 gedrückt ist, und somit feststellt, dass eine Blutkomponente vollständig in den dafür vorgesehenen Komponentenbeutel 13 und 14 gelangt ist. Bei den vorliegenden Modifikationen wird zu diesem Zweck ein Farbsensor verwendet. Der Sensor 17 erkennt folglich eine Farbeänderung, die im Bereich der Öffnung des Blutbeutels 12 auftritt, und ermöglicht es dadurch, dass die Steuereinheit 5 beispielsweise feststellen kann, dass das gelbliche Plasma 16a vollständig aus dem Blutbeutel 12 gedrückt ist. Falls die Steuereinheit 5 unter Verwendung des Sensors 17 feststellt, dass das Plasma 16a vollständig aus dem Blutbeutel 12 gedrückt ist, schaltet sie die zweite Klemme um, so dass die Leitung 14a geöffnet und die Leitung 13a geschlossen ist. Die erste Klemme, durch die die Leitung 12a geführt ist, bleibt weiterhin geöffnet, so dass ein Fließweg von der Öffnung des Blutbeutels 12 über die Leitungen 12a und 14a zu der Öffnung des zweiten Komponentenbeutels 14 vorhanden ist.

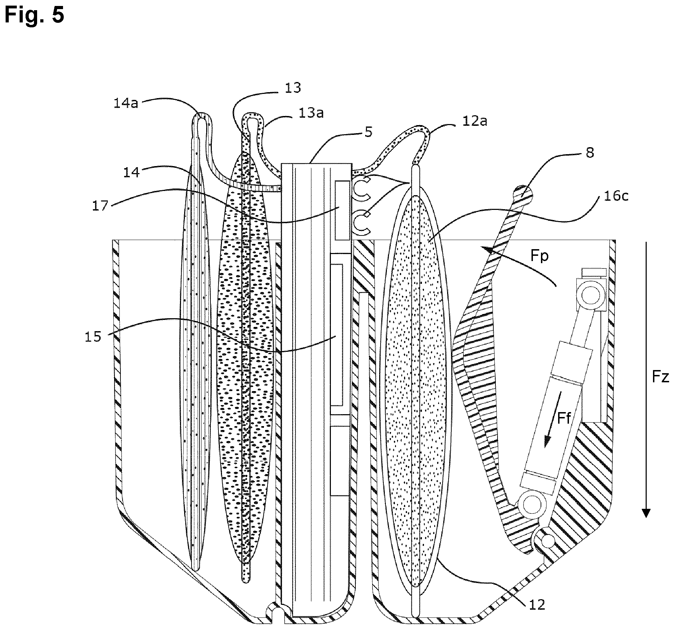

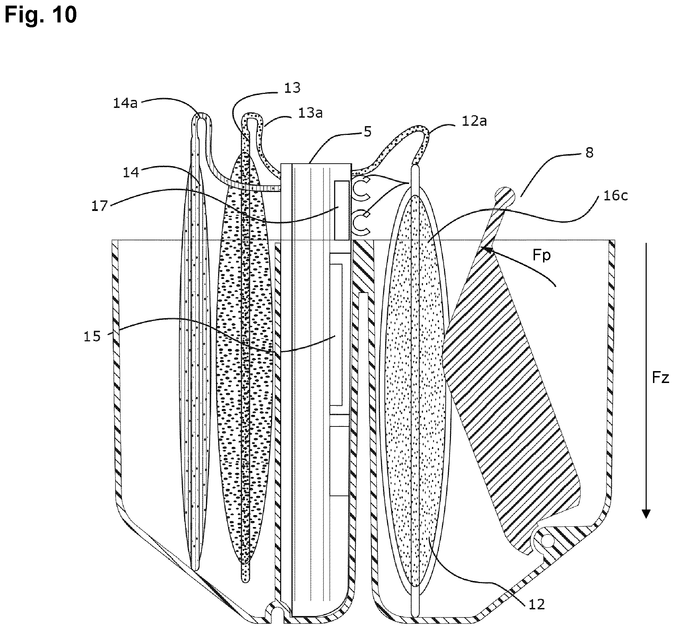

Aufgrund der während der zweiten Phase weiterhin vorhandenen Zentrifugalkraft Fz und/oder der Federkraft Ff wird weiter die zur Richtung der Zentrifugalkraft Fz im Wesentlichen senkrechte Presskraft Fp erzeugt, die auf den Blutbeutel 12 drückt, und der Buffy-Coat 16b wird, wie in den Figuren 5 und 10 zu sehen, aus dem Blutbeutel 12 gedrückt und gelangt auf diese Weise in den zweiten Komponentenbeutel 14. Wenn die Steuereinheit 5 mit Hilfe des Sensors 17 feststellt, dass der Buffy-Coat vollständig in den dafür vorgesehenen Komponentenbeutel 13 gelangt ist, schließt sie das erste Ventil, so dass die Leitung 12a unterbrochen ist. Demzufolge werden die Erythrozyten 16a nicht mehr aus dem Blutbeutel 12 gedrückt und die unterschiedlichen Blutkomponenten 16a, 16b und 16c befinden sich im Blutbeutel 12 sowie den vorgesehenen Komponentenbeuteln 13 und 14 und können einzeln weiterverarbeitet oder untersucht werden.

Es ist nicht zwingend erforderlich, dass der Sensor 17 verwendet wird, um zu bestimmen, dass das Plasma 16a bzw. der Buffy-Coat 16b vollständig aus dem Blutbeutel 12 gedrückt ist. Alternativ kann die Steuereinheit 5 auch die Menge der aus dem Blutbeutel 12 ausgedrückten separierten Blutkomponenten 16a und 16b jeweils mit Hilfe einer empirisch ermittelten Zeitdauer t1 bestimmen. Die Zeitdauer t1 ist dabei für die einzelnen Blutkomponenten 16a bzw. 16b separat festzulegen. Bevorzugt wird die Zentrifuge dazu so eingestellt, dass eine definierte Zentrifugalkraft Fz vorliegt. Die Zeitdauer t1 kann auf diese Weise unabhängig vom Typ der Zentrifuge, d.h. unabhängig von einem Rotordurchmesser und einer Drehzahl, festgelegt werden.

Die definierte Zentrifugalkraft Fz wird dabei durch einen an der Blutseparationsvorrichtung 4 angebrachten Beschleunigungssensor erfasst und die Steuereinheit 5 erlangt den Wert der Zentrifugalkraft Fz vom Beschleunigungssensor. Der Beschleunigungssensor ist dazu bevorzugt an der Steuereinheit 5 angebracht. Der an der Blutseparationsvorrichtung 4 angebrachte Beschleunigungssensor stellt somit den Vorteil bereit, dass die definierte Zentrifugalkraft Fz zuverlässig und unabhängig vom Typ der Zentrifuge eingestellt werden kann. Wenn die definierte Zentrifugalkraft Fz während der zweiten Phase des Zentrifugiervorgangs erreicht ist, kann die Steuereinheit 5 ein Überführen der einzelnen Blutkomponenten 16a und 16b in die jeweiligen Komponentenbeutel 13 und 14 automatisch starten.

Zum Einstellen der vorgegebenen Zentrifugalkraft Fz weist die Blutseparationsvorrichtung 4 eine außerhalb der Zentrifuge bereitgestellte Fernsteuereinheit auf, die imstande ist, mit der Steuereinheit 5 eine bidirektionale kabellose Kommunikation durchzuführen. Die Steuereinheit 5 ist dazu ebenfalls mit einer Einheit zum Durchführen einer bidirektionalen kabellosen Kommunikation mit der Fernsteuereinheit ausgestattet. Die Fernsteuereinheit ist bevorzugt ein Smartphone oder ein Tabletcomputer oder eine ähnliche tragbare Einheit und die kabellose Kommunikation wird über eine W-LAN-Verbindung oder dergleichen durchgeführt. Folglich kann der durch den Beschleunigungssensor erfasste Wert der Zentrifugalkraft Fz von der Steuereinheit 5 an die Fernsteuereinheit übertragen werden und die Drehzahl der Zentrifuge kann entsprechend geändert werden, so dass die vorgegebene Zentrifugalkraft Fz auftritt. Die Überführung der Blutkomponenten 16a und 16b kann anschließend, wenn die vorgegebene Zentrifugalkraft Fz wirkt, entweder durch Verwendung der Fernsteuereinheit manuell oder durch die Steuereinheit 5 automatisch gestartet werden kann. Die Verwendung des Beschleunigungssensors und der Fernsteuereinheit ermöglicht es demzufolge, dass die erfindungsgemäße Blutseparationsvorrichtung 4 auch für einfache Zentrifugen ohne Einheit zum Messen der Zentrifugalkraft Fz verwendbar ist.

Nachdem das Plasma 16a und der Buffy-Coat 16b in die entsprechenden Komponentenbeutel 13 bzw. 14 gedrückt sind, werden die Klemmen, vor allem die erste Klemme, geschlossen, um ein weiteres Ausdrücken der Erythrozyten 16c aus dem Blutbeutel 12 zu verhindern.

Nachdem der Rotor der Zentrifuge angehalten ist, kann das Inlet aus dem Aufnahmebehälter genommen werden und der Blutbeutel 12 mit den Erythrozyten 16c sowie die beiden Komponentenbeutel 13 und 14 mit dem Plasma 16a bzw. dem Buffy-Coat 16b können aus dem Inlet genommen werden.

Demzufolge bietet die vorliegende Erfindung den Vorteil, dass die einzelnen Blutkomponenten 16a, 16b und 16c beim Stillstand der Zentrifuge bereits in getrennten Behältnissen zur Verfügung stehen. Gegenüber konventionellen Arbeitsverfahren, bei denen die Blutkomponenten 16a, 16b und 16c erst nach Auslaufen der Zentrifuge getrennt werden, stellt die erfindungsgemäße Blutseparationsvorrichtung 4 den Vorteil bereit, dass sich die Blutkomponenten 16a, 16b und 16c in der Zeit zwischen dem Zentrifugieren und dem Ausdrücken der einzelnen Blutkomponenten 16a, 16b und 16c unter Verwendung einer separaten Vorrichtung nicht vermischt werden. Zudem sind, verglichen mit einem Arbeitsverfahren, bei dem die Blutkomponenten 16a, 16b und 16c durch eine separate Vorrichtung getrennt werden, weniger Arbeitsschritte erforderlich, wodurch eine Zeitersparnis erreicht wird.

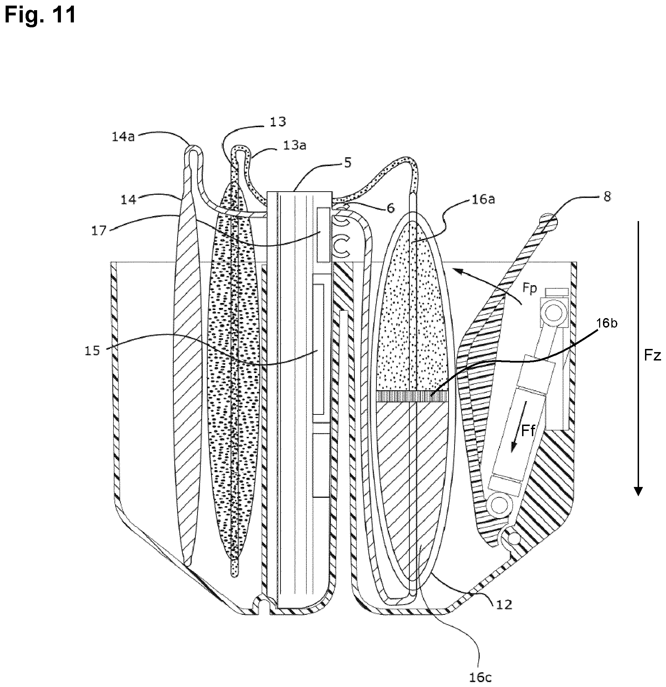

Im Folgenden wird eine Verwendung der erfindungsgemäßen Blutseparationsvorrichtung 4 gemäß einer anderen Ausführungsform beschrieben. Eine der Modifikation der Blutseparationsvorrichtung 4 bei Verwendung gemäß der anderen Ausführungsform ist beispielhaft in Fig. 11 gezeigt. Es ist jedoch festzuhalten, dass jede andere Modifikation der erfindungsgemäßen Blutseparationsvorrichtung 4 gemäß dieser Ausführungsform verwendet werden kann. In dieser Ausführungsform sind der Blutbeutel 12 und die Komponentenbeutel 13 und 14 gemäß einer sog. Top-Bottom-Spezifikation angeordnet. Das bedeutet, dass die beiden Komponentenbeutel 13 und 14 an unterschiedlichen Enden bzw. an einem oberen Ende und einem unteren Ende des Blutbeutels 12 angeschlossen sind. Der Blutbeutel 12 weist daher zwei Öffnungen auf, an denen die Leitungen 13a bzw. 14a direkt angeschlossen sind. Die Leitung 12a und die Y-Abzweigung werden bei dieser Konfiguration somit nicht benötigt. In den Leitungen 13a und 14a ist jeweils ein Brechventil angeordnet, so dass der Blutbeutel 12 in einem Ausgangszustand sicher verschlossen ist.

Der Blutbeutel 12 und die Komponentenbeutel 13 und 14 werden, wie in Fig. 11 gezeigt, vor dem Zentrifugieren in das Inlet eingebracht, wobei der Blutbeutel 12 in der ersten Kammer 4a und die beiden Komponentenbeutel 13 und 14 in der zweiten Kammer 4b angeordnet sind. Bei dieser Ausführungsform ist, wie bereits erwähnt, eine Öffnung des Blutbeutels 12, die mit dem Komponentenbeutel 13 über die eine Leitung 13a verbunden ist, oben und eine andere Öffnung des Blutbeutels 12, die mit dem Komponentenbeutel 14 über die andere Leitung 14a verbunden ist, unten angeordnet. Um die Verteilung der Blutkomponenten 16a, 16b und 16c zu steuern, werden die Leitungen 13a bzw. 14a in eine erste Klemme bzw. eine zweite Klemme eingebracht, die einen Fließweg in den Leitungen 13a und 14a vor einem Zentrifugieren unterbrechen. Anschließend können die beiden Brechventile in den Leitungen 13a und 14a geöffnet werden und das Inlet kann in den Aufnahmebehälter der Zentrifuge eingebracht werden. Wie oben beschrieben, können die Brechventile auch erst nach Einbringen des Inlets in den Aufnahmebehälter geöffnet werden.

Wie in der vorhergehend beschriebenen Ausführungsform wird das Blut während der ersten Phase des Zentrifugiervorgangs in die einzelnen Blutkomponenten, Plasma 16a, Buffy-Coat 16b und Erythrozyten 16c, aufgetrennt.

Insbesondere eine Aufteilung der einzelnen Komponenten 16a, 16b, 16c auf die Komponentenbeutel 13 und 14 und den Blutbeutel 12 während der zweiten Phase des Zentrifugiervorgangs unterscheidet sich gegenüber der vorhergehend beschriebenen Ausführungsform. Der Verdrängungskörper 8 drückt mit Hilfe der Zentrifugalkraft Fz und/oder der Federkraft Ff auf den Blutbeutel 12 und es werden nacheinander die erste Klemme, in die die Leitung 13a eingebracht ist, und die zweite Klemme, in die die Leitung 14a eingebracht ist, geöffnet. Auf diese Weise gelangt zuerst das Plasma 16a durch die Öffnung am oberen Ende des Blutbeutels 12 über die Leitung 13a in den Komponentenbeutel 13 und anschließend gelangen die Erythrozyten 16c durch die Öffnung am unteren Ende des Blutbeutels 12 über die andere Leitung 14a in den anderen Komponentenbeutel 14. Es ist auch möglich, zuerst die Erythrozyten 16c und dann das Plasma 16a in die entsprechenden Komponentenbeutel 13 bzw. 14 zu überführen. Im Gegensatz zur Verwendung gemäß der ersten Ausführungsform verbleibt der Buffy-Coat 16b im Blutbeutel 12 und die Erythrozyten 16c werden in den Komponentenbeutel 14 transportiert. In dieser Ausführungsform wird mit Hilfe von Sensoren 17 bestimmt wird, ob das Plasma 16a und die Erythrozyten 16c vollständig aus dem Blutbeutel 12 gedrückt sind. Zu diesem Zweck ist zusätzlich zum Sensor 17 im Bereich der oberen Öffnung des Blutbeutels 12 ein weiterer Sensor 17 im Bereich der unteren Öffnung des Blutbeutels 12 anzuordnen. Alternativ dazu kann wieder eine Zeitdauer t1 bestimmt werden, die benötigt wird, um das Plasma 16a bzw. die Erythrozyten 16c aus dem Blutbeutel zu drücken. Die Zeitdauer t1 ist dabei für die einzelnen Blutkomponenten 16a bzw. 16c separat festzulegen. Bevorzugt wird die Zeitdauer t1 wieder für eine vorgegebene Zentrifugalkraft Fz bestimmt, die während der zweiten Phase des Zentrifugiervorgangs mit Hilfe des Beschleunigungssensors bestimmt wird.

Bevorzugt steuert die Steuereinheit 5 die erste Klemme und die zweite Klemme abwechselnd, um den Buffy-Coat 16b in einem vorgegebenen Bereich des Blutbeutels 12 zu halten. Die Lage des Buffy-Coat 16b wird dabei mit Hilfe des oben beschriebenen Lage-Sensors ermittelt. Auf diese Weise wird verhindert, dass der Buffy-Coat 16b von der Position, an der er nach Beendigung des Zentrifugiervorgangs angeordnet ist, bis zu einer der beiden Öffnungen gedrückt wird. Dadurch können Verunreinigungen mit Buffy-Coat 16b an der Innenwand des Blutbeutels 12, die anschließend im Plasma 16a und/oder den Erythrozyten 16c vorhanden sind, verglichen mit einem Fall reduziert werden, in dem der Buffy-Coat 16b bei vollständigem Ausdrücken einer der beiden Blutkomponenten 16a und 16c bis in den Bereich der Öffnungen am oberen bzw. unteren Ende des Blutbeutels 12 gelangt. Dieses Vorgehen, bei dem der Blutbeutel 12 und die Komponentenbeutel 13, 14 gemäß einer Top-Bottom-Anordnung verwendet und das Plasma 16a und die Erythrozyten 16c durch abwechselndes Öffnen der Klemmen ausgedrückt werden, bietet somit den Vorteil, dass nicht zwingend ein Leukozytenfilter erforderlich ist, da Verunreinigungen durch den Buffy-Coat 16b reduziert werden. Nachdem das Plasma 16a und die Erythrozyten 16c in die entsprechenden Beutel 13 bzw. 14 gedrückt sind, werden die Klemmen, in denen die Leitungen 13a und 14a aufgenommen sind, geschlossen, um ein Ausdrücken des Buffy-Coat 16b aus dem Blutbeutel 12 zu verhindern.

Nachdem der Rotor der Zentrifuge angehalten ist, kann das Inlet aus dem dafür vorgesehenen Aufnahmebehälter genommen werden und die beiden Komponentenbeutel 13 und 14, die das Plasma 16a bzw. die Erythrozyten 16c aufweisen, und der Blutbeutel 12, der den Buffy-Coat 16b aufweist, können aus dem Inlet genommen werden. Wie die obige Ausführungsform stellt diese Ausführungsform den Vorteil bereit, dass die Komponenten nach Beendigung des Zentrifugiervorgangs auf die einzelnen Komponentenbeutel 13 und 14 und den Blutbeutel 12 aufgeteilt sind. Somit wird ein erneutes Vermischen der Blutkomponenten 16a, 16b, 16c in der Zeit bis zu einem Einbringen in eine externe Separationsvorrichtung vermieden.

## Patentansprüche

1. Blutseparationsvorrichtung (4) zum Separieren von Blut (16) in mehrere Blutkomponenten (16a; 16b; 16c) unter Einfluss einer während eines Zentrifugiervorgangs in einer Zentrifuge erzeugten Zentrifugalkraft (Fz), mit:
einer ersten Kammer (4a) zur Aufnahme eines Blutbeutels (12), der mit dem zu separierenden Blut (16) gefüllt ist, das sich unter Einfluss der Zentrifugalkraft (Fz) in die einzelnen Blutkomponenten (16a; 16b; 16c) derart auftrennt, dass sich jede Blutkomponente (16a; 16b; 16c) nach Beendigung einer ersten Phase des Zentrifugiervorgangs - in Richtung der Zentrifugalkraft (Fz, "Separationsrichtung") gesehen - entsprechend ihrem spezifischen Gewicht in einem separaten Abschnitt des Blutbeutels (12) befindet,
einer zweiten Kammer (4b) zur Aufnahme mindestens eines Komponentenbeutels (13; 14), der zur Befüllung mit einer jeweils zugeordneten Blutkomponente (16a; 16b; 16c) vorgesehen ist,
wobei eine Einlassöffnung jedes Komponentenbeutels (13; 14) über eine Leitung (12a; 13a; 14a), in der ein steuerbares Ventil angeordnet ist, mit einer Ausgangsöffnung des Blutbeutels (12) verbunden ist,
einem Verdrängungskörper (8), der in der ersten Kammer (4a) angeordnet ist und auf den Blutbeutel (12) eine im Wesentlichen senkrecht zur Separationsrichtung gerichtete Presskraft (Fp) ausübt, wobei der Verdrängungsköper (8) so geformt ist, dass er die Presskraft (Fp) mit Hilfe der Zentrifugalkraft (Fz) und/oder mit Hilfe einer Federkraft (Ff) erzeugt, und
einer Steuereinheit (5), die während einer zweiten Phase des Zentrifugiervorgangs jedes Ventil so lange öffnet, bis die diesem Ventil zugeordnete Blutkomponente (16a; 16b; 16c) infolge der Presskraft (Fp) des Verdrängungskörpers (8) in den zugeordneten Komponentenbeutel (13; 14) gelangt ist,
wobei
der Verdrängungsköper (8) eine Hebelanordnung aufweist, die einen Teil der Zentrifugalkraft (Fz) und/oder der Federkraft (Ff) so umlenkt, dass sie die Presskraft (Fp) erzeugt.

2. Blutseparationsvorrichtung (4) nach Anspruch 1, **dadurch gekennzeichnet, dass** die Hebelanordnung an einer Wandung der ersten Kammer (4a) an einer Rotationsachse (11) drehbar angebracht ist.

3. Blutseparationsvorrichtung (4) nach Anspruch 2, **dadurch gekennzeichnet, dass** zur Erzeugung der Federkraft (Ff) ein Ende einer Feder (9) an der Wandung der ersten Kammer (4a) und ein anderes Ende der Feder (9) an der Hebelanordnung angebracht ist.

4. Blutseparationsvorrichtung (4) nach einem der Ansprüche 1 bis 3, **dadurch gekennzeichnet, dass** ein oberer Abschnitt der Hebelanordnung in einem vorgegebenen Winkel in Richtung der Wandung abgewinkelt ist.

5. Blutseparationsvorrichtung (4) nach einem der Ansprüche 1 bis 4, **dadurch gekennzeichnet, dass** an der Hebelanordnung eine kardanische Lagerung (8b) und einer Pressplatte (8c) angebracht sind.

6. Blutseparationsvorrichtung (4) nach einem der Ansprüche 1 bis 5, **dadurch gekennzeichnet, dass** die Steuereinheit (5) zur Erfassung der Menge der aus dem Blutbeutel (12) gerade ausgedrückten separierten Blutkomponente (16a; 16b; 16c) mindestens einen Sensor (17) aufweist.

7. Blutseparationsvorrichtung (4) nach einem der Ansprüche 1 bis 6, **dadurch gekennzeichnet, dass** die Steuereinheit (5) die Menge der aus dem Blutbeutel (12) gerade ausgedrückten separierten Blutkomponente (16a; 16b; 16c) jeweils aus einer empirisch ermittelten Zeitdauer (t1) bestimmt.

8. Blutseparationsvorrichtung (4) nach einem der Ansprüche 1 bis 7, **gekennzeichnet durch** einen Beschleunigungssensor zum Erfassen der Zentrifugalkraft (Fz).

9. Blutseparationsvorrichtung (4) nach einem der Ansprüche 1 bis 8,
**gekennzeichnet durch** eine Fernsteuereinheit zum Durchführen einer bidirektionalen Kommunikation mit der Steuereinheit (5).

10. Blutseparationsvorrichtung (4) nach einem der vorangehenden Ansprüche,
**dadurch gekennzeichnet, dass** die steuerbaren Ventile elektromotorisch angetriebene Klemmen sind.

11. Blutseparationsvorrichtung (4) nach einem der vorangehenden Ansprüche,
**dadurch gekennzeichnet, dass** die beiden Kammern (4a; 4b) sowie die Steuereinheit (5) in einem offenen Behälter ("Inlet") angeordnet sind, dessen Außengestalt an die Innenform eines Aufnahmebehälters einer dafür vorgesehenen Zentrifuge angepasst ist.

12. Blutseparationsvorrichtung (4) nach Anspruch 11, **dadurch gekennzeichnet, dass** das Inlet entnehmbar ausgebildet ist, so dass der Blutbeutel (12) sowie die Komponentenbeutel (13; 14) im ausgebauten Zustand des Inlets in das Inlet einsetzbar bzw. nach Beendigung der Blutseparation wieder herausnehmbar sind.

13. Blutseparationsvorrichtung (4) nach einem der vorangehenden Ansprüche,
**gekennzeichnet durch** eine Führungsplatte (18) zum Führen der Leitungen (12a; 13a; 14a).

## Claims

1. A blood separation device (4) for separating blood (16) into a plurality of blood components (16a; 16b; 16c) under the influence of a centrifugal force (Fz) generated during a centrifugation process in a centrifuge, comprising:
a first chamber (4a) for receiving a blood bag (12) filled with the blood (16) to be separated, which separates into the individual blood components (16a; 16b; 16c) under the influence of the centrifugal force (Fz) in such a way that each blood component (16a; 16b; 16c) is located - as seen in the direction of the centrifugal force (Fz, 'separation direction') - in a separate section of the blood bag (12) according to its specific weight after completion of a first phase of the centrifuging process,
a second chamber (4b) for receiving at least one component bag (13; 14) which is provided for filling with a respectively associated blood component (16a; 16b; 16c),
wherein an inlet opening of each component bag (13; 14) is connected via a line (12a; 13a; 14a), in which a controllable valve is arranged, is connected to an outlet opening of the blood bag (12),
a displacement body (8), which is arranged in the first chamber (4a) and exerts a pressing force (Fp) onto the blood bag (12) which is directed substantially perpendicular to the direction of separation, wherein the displacement body (8) is shaped in order to generate the pressing force (Fp) by means of the centrifugal force (Fz) and/or by means of a spring force (Ff), and
a control unit (5), which during a second phase of the centrifuging process opens each valve until the blood component (16a; 16b; 16c) associated with this valve has passed into the associated component bag (13; 14) as a result of the pressing force (Fp) of the displacement body (8),
wherein
the displacement body (8) has a lever arrangement which redirects part of the centrifugal force (Fz) and/or of the spring force (Ff) so that it generates the pressing force (Fp).

2. The blood separation device (4) in accordance with claim 1, **characterised in that** the lever arrangement is rotatably mounted on a wall of the first chamber (4a) on an axis of rotation (11).

3. The blood separation device (4) in accordance with claim 2, **characterised in that** for generating the spring force (Ff) one end of a spring (9) is attached to the wall of the first chamber (4a) and another end of the spring (9) is attached to the lever arrangement.

4. The blood separation device (4) in accordance with any one of claims 1 to 3, **characterised in that** an upper portion of the lever assembly is angled at a predetermined angle towards the wall.

5. The blood separation device (4) in accordance with any one of claims 1 to 4, **characterised in that** a gimbal bearing (8b) and a pressing plate (8c) are attached to the lever arrangement.

6. The blood separation device (4) in accordance with any one of claims 1 to 5, **characterised in that** the control unit (5) comprises at least one sensor (17) for detecting the amount of separated blood component (16a; 16b; 16c) just ejected out of the blood bag (12).

7. The blood separation device (4) according to any one of claims 1 to 6, **characterised in that** the control unit (5) is used to determine the amount of separated blood components (16a, 16b, 16c) that are ejected out of the blood bag (12) wherein, in each case an empirically determined time duration (t1) is used

8. The blood separation device (4) in accordance with any one of claims 1 to 7, **characterised by** an acceleration sensor for detecting the centrifugal force (Fz).

9. The blood separation device (4) in accordance with any one of claims 1 to 8, **characterised by** a remote control unit for performing bidirectional communication with the control unit (5).

10. The blood separation device (4) in accordance with any one of the preceding claims, **characterised in that** the controllable valves are electric motor driven clamps.

11. The blood separation device (4) in accordance with any one of the preceding claims, **characterised in that** the two chambers (4a; 4b) as well as the control unit (5) are arranged in an open container ('inlet'), the outer shape of which is adapted to the inner shape of a receiving container of a centrifuge which is provided for this.

12. The blood separation device (4) in accordance with claim 11, **characterised in that** the inlet is designed to be removable so that the blood bag (12) and the component bags (13; 14) can be inserted into the inlet when the inlet is removed and/or can be removed again when blood separation is complete.

13. The blood separation device (4) in accordance with any one of the preceding claims, 25 **characterised by** a guide plate (18) for guiding the lines (12a; 13a; 14a).

## Revendications

1. Dispositif séparateur de sang (4) pour la séparation de sang (16) en plusieurs composants sanguins (16a ; 16b ; 16c) sous l'influence d'une force centrifuge (Fz) générée pendant un processus de centrifugation dans une centrifugeuse, avec :
une première chambre (4a) pour la réception d'une poche de sang (12) qui est remplie avec le sang à séparer (16) qui se sépare sous l'influence de la force centrifuge (Fz) en les composants sanguins (16a ; 16b ; 16c) individuels de telle manière que chaque composant sanguin (16a ; 16b ; 16c) se trouve à la fin d'une première phase du processus de centrifugation - vu en direction de la force centrifuge (Fz "sens de séparation") - selon son poids spécifique dans une section séparée de la poche de sang (12),
une seconde chambre (4b) pour la réception d'au moins une poche de composant (13 ; 14) qui est prévue pour le remplissage avec un composant sanguin (16a ; 16b ; 16c) associé respectivement,
dans lequel une ouverture d'entrée de chaque poche de composant (13 ; 14) est reliée par le biais d'une conduite (12a ; 13a ; 14a), dans laquelle une vanne commandable est agencée à une ouverture de sortie de la poche de sang (12),
un corps de refoulement (8) qui est agencé dans la première chambre (4a) et exerce sur la poche de sang (12) une force de pressage (Fp) dirigée sensiblement perpendiculairement au sens de séparation, dans lequel le corps de refoulement (8) est formé de sorte qu'il génère la force de pressage (Fp) à l'aide de la force centrifuge (Fz) et/ou à l'aide d'une force de ressort (Ff), et
un dispositif de commande (5) qui ouvre pendant une seconde phase du processus de centrifugation chaque vanne jusqu'à ce que le composant sanguin (16a ; 16b ; 16c) associé à cette vanne soit parvenu à la suite de la force de pressage (Fp) du corps de refoulement (8) dans la poche de composant (13 ; 14) associée,
dans lequel
le corps de refoulement (8) présente un agencement de levier qui renvoie une partie de la force centrifuge (Fz) et/ou de la force de ressort (Ff) de sorte qu'il génère la force de pressage (Fp).

2. Dispositif séparateur de sang (4) selon la revendication 1, **caractérisé en ce que** l'agencement de levier est monté de manière rotative au niveau d'une paroi de la première chambre (4a) sur un axe de rotation (11).

3. Dispositif séparateur de sang (4) selon la revendication 1, **caractérisé en ce que** pour la génération de la force de ressort (Ff), une extrémité d'un ressort (9) est montée au niveau de la paroi de la première chambre (4a) et une autre extrémité du ressort (9) au niveau de l'agencement de levier.

4. Dispositif séparateur de sang (4) selon l'une quelconque des revendications 1 à 3, **caractérisé en ce qu'**une section supérieure de l'agencement de levier est coudée dans un angle prédéfini en direction de la paroi.

5. Dispositif séparateur de sang (4) selon l'une quelconque des revendications 1 à 4, **caractérisé en ce qu'**un logement (8b) de cardan et une plaque de pressage (8c) sont montés au niveau de l'agencement de levier.

6. Dispositif séparateur de sang (4) selon l'une quelconque des revendications 1 à 5, **caractérisé en ce que** le dispositif de commande (5) présente pour la détection de la quantité du composant sanguin (16a ; 16b ; 16c) séparé éjecté justement de la poche de sang (12) au moins un capteur (17).

7. Dispositif séparateur de sang (4) selon l'une quelconque des revendications 1 à 6, **caractérisé en ce que** le dispositif de commande (5) détermine la quantité du composant sanguin (16a ; 16b ; 16c) séparé éjecté justement de la poche de sang (12) respectivement à partir d'une durée déterminée par voie empirique.

8. Dispositif séparateur de sang (4) selon l'une quelconque des revendications 1 à 7, **caractérisé par** un capteur d'accélération pour la détection de la force centrifuge (Fz).

9. Dispositif séparateur de sang (4) selon l'une quelconque des revendications 1 à 8, **caractérisé par** une unité de télécommande pour la réalisation d'une communication bidirectionnelle avec le dispositif de commande (5).

10. Dispositif séparateur de sang (4) selon l'une quelconque des revendications précédentes, **caractérisé en ce que** les vannes commandables sont des pinces entraînées par voie électromotorisée.

11. Dispositif séparateur de sang (4) selon l'une quelconque des revendications précédentes, **caractérisé en ce que** les deux chambres (4a ; 4b) ainsi que le dispositif de commande (5) sont agencés dans un récipient ouvert ("entrée"), dont la forme extérieure est adaptée à la forme intérieure d'un récipient de réception d'une centrifugeuse prévue à cet effet.

12. Dispositif séparateur de sang (4) selon la revendication 11, **caractérisé en ce que** l'entrée est réalisée de manière retirable de sorte que la poche de sang (12) ainsi que la poche de composant (13 ; 14) puissent être insérées dans l'état démonté de l'entrée dans l'entrée ou de nouveau retirées à la fin de la séparation du sang.

13. Dispositif séparateur de sang (4) selon l'une quelconque des revendications précédentes, **caractérisé par** une plaque de guidage (18) pour le guidage des conduites (12a ; 13a ; 14a).
